(19) Europäisches Patentamt
European Patent Office
Office européen des brevets

(11) **EP 4 303 284 A1**

(12) **EUROPEAN PATENT APPLICATION**
published in accordance with Art. 153(4) EPC

(43) Date of publication:
**10.01.2024 Bulletin 2024/02**

(21) Application number: **22762821.1**

(22) Date of filing: **20.01.2022**

(51) International Patent Classification (IPC):
*C09K 23/00* (2022.01)    *A23L 27/60* (2016.01)
*A23C 11/00* (2006.01)    *A23L 5/00* (2016.01)
*A23L 29/10* (2016.01)    *A23L 33/145* (2016.01)

(52) Cooperative Patent Classification (CPC):
**A23C 11/00; A23L 5/00; A23L 27/60; A23L 29/00;
A23L 29/10; A23L 33/145; C09K 23/00**

(86) International application number:
**PCT/JP2022/002006**

(87) International publication number:
**WO 2022/185762 (09.09.2022 Gazette 2022/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.03.2021 JP 2021035500**

(71) Applicants:
• **Asahi Group Foods, Ltd.**
  **Tokyo, 130-8602 (JP)**
• **Asahi Group Holdings, Ltd.**
  **Tokyo 130-8602 (JP)**

(72) Inventors:
• **KANO Takahiro**
  **Tokyo 150-0022 (JP)**
• **OTSUSHI Noboru**
  **Tokyo 150-0022 (JP)**
• **TANAKA Masayuki**
  **Tokyo 150-0022 (JP)**
• **TANAKA Takeshi**
  **Tokyo 150-0022 (JP)**

(74) Representative: **Hoffmann Eitle**
**Patent- und Rechtsanwälte PartmbB**
**Arabellastraße 30**
**81925 München (DE)**

(54) **COMPOSITION CONTAINING DECOMPOSITION PRODUCT OF YEAST CELL WALL, METHOD FOR PRODUCING SAID COMPOSITION, AND USE OF SAID COMPOSITION**

(57)    This is to provide a composition containing a decomposition product of yeast cell body residue useful as an emulsifier substitute, an emulsion stabilizer substitute or a milk-like substitute, and a method for producing the same, and use thereof.

Provided is a composition containing a cell wall lysing enzyme-decomposition product of yeast cell body residue, wherein the decomposition product contains 5% by mass or more of lipid based on a dry mass of the decomposition product.

EP 4 303 284 A1

**Description**

TECHNICAL FIELD

The present invention relates to a composition containing a decomposition product of a yeast cell wall useful as an emulsifier substitute, an emulsion stabilizer substitute or a milk-like substitute and a method for producing the same, and use thereof.

BACKGROUND ART

In recent years, in order to avoid allergens derived from milk, from an increase in demand for low-fat or non-fat foods due to diet consciousness and health consciousness, or from an increase in demand for vegan foods, etc., more attention has been paid to foods using a milk-like substitute derived from plants such as oats, soybeans, almonds, etc., in place of a milk raw material. However, these milk-like substitutes derived from plants are usually more expensive than cow's milk and are known to be foods containing allergic substances.

Similarly, in addition to synthetic emulsifiers, natural product-derived emulsifiers and emulsion stabilizers such as egg-derived lecithin and milk-derived casein are used as surfactants in foods, but these can also be allergens. In addition, these emulsifiers are subject to specifications as food additives under the Food Sanitation Law, and must be labeled as food additives when used in food products. Therefore, emulsifiers for foods in which yeast extract is an effective ingredient have been reported as emulsifiers derived from natural products, which do not require indication of food additives, and which are allergen-free (for example, see Patent Document 1).

PRIOR ART DOCUMENT

PATENT DOCUMENT

Patent Document 1: JP 2012-231747A

SUMMARY OF THE INVENTION

PROBLEMS TO BE SOLVED BY THE INVENTION

An object of the present invention is to provide a composition useful as an emulsifier substitute, an emulsion stabilizer substitute or a milk-like substitute and a method for producing the same, and use thereof.

MEANS TO SOLVE THE PROBLEMS

The present inventors have earnestly studied in view of the above-mentioned problems, and found that a composition containing a cell wall lysing enzyme-decomposition product of yeast cell body residue is useful as an emulsifier substitute, an emulsion stabilizer substitute or a milk-like substitute, whereby they have completed the present invention.

Accordingly, the gist of the present invention is as follows.

[1] A composition containing a decomposition product of a yeast cell wall which contains a cell wall lysing enzyme-decomposition product of yeast cell body residue.

[2] A composition containing a decomposition product of a yeast cell wall containing a cell wall lysing enzyme-decomposition product of yeast cell body residue, wherein the decomposition product contains 5% by mass or more of lipid based on a dry mass of the decomposition product.

[3] The composition described in [1] or [2], wherein an RNA content of the decomposition product is less than 5% by mass based on a dry mass of the decomposition product.

[4] The composition described in any of [1] to [3], wherein the decomposition product contains an insoluble peptide.

[5] The composition described in any of [1] to [4], wherein the cell wall lysing enzyme is glucanase.

[6] The composition described in any of [1] to [5], wherein the cell wall lysing enzyme is glucanase having $\beta$-1,3, $\beta$-1,4, and/or $\beta$-1,6 activity.

[7] The composition described in any of [1] to [6], wherein the cell wall lysing enzyme is glucanase derived from Streptomyces.

[8] The composition described in any of [1] to [7], wherein the cell wall lysing enzyme is glucanase having no protease activity.

[9] The composition described in any of [1] to [8], wherein the lipid contains at least yeast-derived phospholipid.

[10] The composition described in any of [1] to [9], wherein the decomposition product contains 5% by mass or more and 15% by mass or less of lipid based on a dry mass of the decomposition product.

[11] The composition described in any of [1] to [10], wherein the decomposition product is a material obtained by the method containing the following:

(a) treating a yeast cell body residue with glucanase at 40 to 60°C for 1 to 24 hours, and
(b) recovering the treated product obtained in (a).

[12] The composition described in any of [1] to [11], wherein the yeast cell body residue is a residue of the yeast cell body after extraction of yeast with hot water.

[13] A method for producing a composition containing a decomposition product of a yeast cell wall, which contains

(A) treating a yeast cell body residue with a cell wall lysing enzyme, and
(B) recovering the treated product obtained in (A).

[14] An emulsifier for food or an emulsion stabilizer for food which contains the composition described in any of [1] to [12].

[15] A food composition which contains the composition described in any of [1] to [12], oils and fats and carbohydrates.

[16] Use of the composition described in any of [1] to [12] as an emulsifier for food or an emulsion stabilizer for food.

[17] Use of the composition described in any of [1] to [12] as a milk-like substitute.

## EFFECTS OF THE INVENTION

According to the present invention, it is possible to provide a composition containing a decomposition product of a yeast cell wall useful as an emulsifier substitute, an emulsion stabilizer substitute or a milk-like substitute using a yeast cell body residue which is inexpensive and easily available as a raw material. These compositions are derived from non-animal and non-plant raw materials, do not require indication of food additives, and are free from allergens such as milk, eggs and legumes, so that it can be expected to be used, as an emulsifier or an emulsion stabilizer, or as a milk-like substitute, for various kinds of foods, cosmetics and quasi-drugs, etc.

## EMBODIMENTS TO CARRY OUT THE INVENTION

In one embodiment of the present invention, it is provided a composition containing a cell wall lysing enzyme-decomposition product of yeast cell body residue, and preferably a composition in which the decomposition product contains 5% by mass or more of lipid based on a dry mass of the decomposition product. Also, in another embodiment, it is provided a composition in which the decomposition product contains insoluble peptides, and in yet another embodiment, it is provided a composition in which an RNA content of the decomposition product is less than 5% by mass based on a dry mass of the decomposition product.

In another embodiment of the present invention, it is provided a cell wall lysing enzyme-decomposition product of yeast cell body residue which is a cell wall lysing enzyme-decomposition product of yeast cell body residue in which the decomposition product preferably contains 5% by mass or more of lipid based on a dry mass of the decomposition product. In addition, in another embodiment, it is provided a cell wall lysing enzyme-decomposition product of yeast cell body residue containing an insoluble peptide, and in yet another embodiment, it is provided a cell wall lysing enzyme-decomposition product of yeast cell body residue in which an RNA content is less than 5% by mass based on a dry mass of the decomposition product.

In another embodiment of the present invention, it is provided use of a composition containing a cell wall lysing enzyme-decomposition product of yeast cell body residue as an emulsifier or an emulsion stabilizer for food, or as a milk-like substitute, which is preferably use of the composition in which the decomposition product contains 5% by mass or more of lipid based on a dry mass of the decomposition product. In addition, in another embodiment, it is provided use wherein the composition in such uses contains an insoluble peptide, and in yet another embodiment, the composition in such uses is a material in which an RNA content is less than 5% by mass based on a dry mass of the decomposition product.

In another embodiment of the present invention, it is provided a food composition containing a cell wall lysing enzyme-decomposition product of yeast cell body residue, which is preferably a composition in which the decomposition product contains 5% by mass or more of lipid based on a dry mass of the decomposition product. In addition, in another embodiment, it is provided a food composition in which the decomposition product contains an insoluble peptide, and in yet another embodiment, it is provided a food composition in which an RNA content of the decomposition product is less than 5% by mass based on a dry mass of the decomposition product.

The terms "yeast cell body residue" used in the present specification are not specifically limited as long as it is a material containing cell wall of yeast and is a residue (insoluble fraction) of yeast cell body obtained after yeast is subjected to extraction treatment. Specifically, it means yeast cell body generating as a residue by subjecting yeast to known extraction treatments such as self-digestion treatment (protease treatment), hot water treatment, acid treatment, alkali treatment, and/or mechanical pulverization treatment, etc., and removing the supernatant (water-soluble fraction (yeast extract)) separated by centrifugation, etc. The yeast cell body residue is preferably a residue (water insoluble fraction) of the yeast cell body after extraction of yeast with hot water.

The yeast is not particularly limited as long as it can be applied to the food field, and for example, there may be mentioned yeast for producing beer, yeast for producing bread, yeast for producing rice wine, etc. Or else, whereas the yeast is not limited to these, there may be mentioned, for example, those belonging to genera such as Saccharomyces, Saccharomycodes, Rhodotorula, Endomycopsis, Nematospora, Brettanomyces, Candida, and Torulopsis, etc. These may be used alone or in combination of two or more kinds.

The "cell wall lysing enzyme" used in the present specification means an enzyme or combination of enzymes capable of decomposing part or all of the cell wall of yeast. The cell wall lysing enzyme is preferably those having endo activity, and more preferably those having endo activity alone. As the cell wall lysing enzyme, those having low, almost no or completely no (that is, having no protease activity) protease activity are preferable. Or else, when an enzyme having protease activity is used as the cell wall lysing enzyme, or when an enzyme having protease activity coexists, it is preferable to use it under conditions (for example, pH, temperature, etc.) that suppress protease activity.

The cell wall lysing enzyme may be, for example, those derived from a natural product, those commercially available, or those obtained by a method using genetic recombination technology, etc.

As the cell wall lysing enzyme, whereas it is not limited to these, there may be mentioned, for example, glucanase, mannanase, etc. The glucanase (preferably glucanase having endo activity (preferably having endo activity alone), and/or protease having no activity) is, for example, glucanase having $\beta$-1,3, $\beta$-1,4, and/or $\beta$-1,6 activity, preferably glucanase derived from the genus Streptomyces or the genus Talaromyces, more preferably glucanase derived from the genus Streptomyces, further preferably glucanase derived from Streptomyces, and having $\beta$-1,3, $\beta$-1,4, and/or $\beta$-1,6 activity, still further preferably glucanase derived from Streptomyces having $\beta$-1,3, $\beta$-1,4, and/or $\beta$-1,6 activity and having endo activity (preferably having endo activity alone), even more preferably glucanase derived from Streptomyces having $\beta$-1,3, $\beta$-1,4, and/or $\beta$-1,6 activity, having endo activity, and having no protease activity (for example, Denazyme GEL1/R available from NAGASE & Co., Ltd., etc.), and even more preferably glucanase derived from Streptomyces having $\beta$-1,3, $\beta$-1,4, and/or $\beta$-1,6 activity, having endo activity alone, and having no protease activity.

In one embodiment of the present invention, the cell wall lysing enzyme is glucanase.

In one embodiment of the present invention, the cell wall lysing enzyme is glucanase having $\beta$-1,3, $\beta$-1,4, and/or $\beta$-1,6 activity.

In one embodiment of the present invention, the cell wall lysing enzyme is glucanase derived from Streptomyces.

In one preferable embodiment of the present invention, the cell wall lysing enzyme is glucanase derived from Streptomyces and having $\beta$-1,3, $\beta$-1,4, and/or $\beta$-1,6 activity.

In one embodiment of the present invention, the cell wall lysing enzyme is glucanase having endo activity (preferably having endo activity alone).

In one preferable embodiment of the present invention, the cell wall lysing enzyme is glucanase derived from Streptomyces having $\beta$-1,3, $\beta$-1,4, and/or $\beta$-1,6 activity and having endo activity (preferably having endo activity alone).

In one embodiment of the present invention, the cell wall lysing enzyme is glucanase having no protease activity.

In one preferable embodiment of the present invention, the cell wall lysing enzyme is glucanase derived from Streptomyces having $\beta$-1,3, $\beta$-1,4, and/or $\beta$-1,6 activity, having endo activity (preferably having endo activity alone), and having no protease activity.

The "yeast cell wall decomposition product" used in the present specification means those obtained by decomposing a yeast cell body residue with a cell wall lysing enzyme, and specifically those obtained by decomposing a yeast cell wall contained in a yeast cell body residue with a cell wall lysing enzyme.

As the method for obtaining the "yeast cell wall decomposition product", whereas it is not limited to these, there may be mentioned, for example, methods known to those skilled in the art, methods described in Examples, or the method containing the following:

    (a) treating a yeast cell body residue with a cell wall lysing enzyme, and
    (b) recovering the treated product obtained in (a),

or methods similar to these.

The "yeast cell body residue" in the above-mentioned (a) is as mentioned above, and is preferably a residue of yeast cell body after extraction of yeast with hot water.

The "cell wall lysing enzyme" in the above-mentioned (a) is as mentioned above, and is preferably glucanase.

The "treatment" in the above-mentioned (a) is not particularly limited as long as the conditions are such that the cell wall lysing enzyme can decompose the yeast cell wall contained in the yeast cell body residue, and can be appropriately changed according to the origin of the yeast cell wall, the kind and/or the amount of the cell wall lysing enzyme, and characteristics to be desired, etc. The treatment is usually carried out in a desired solvent (for example, water). For example, it is carried out using a suspension in which the yeast cell body residue is suspended in a solvent (for example, water). The suspension may be, if necessary, applied to a sterilization treatment (for example, heat sterilization, filtration sterilization, etc.). The treatment of the yeast cell body residue with the cell wall lysing enzyme can be carried out, for example, at exceeding 0 to lower than about 100°C (preferably about 10 to about 70°C, more preferably about 25 to about 65°C, and further preferably about 40 to about 60°C), for about 0.5 to about 120 hours (preferably about 0.5 to about 60 hours, more preferably about 1 to about 24 hours, further preferably about 3 hours to about 24 hours, and still further preferably about 12 to about 24 hours) and a pH of about 1 to about 12 (preferably about 2 to about 10, more preferably about 3 to about 8, and further preferably about 4 to about 6).

After the above-mentioned treatment, the cell wall lysing enzyme may be deactivated by high temperature treatment, acid or alkali treatment, etc., if necessary.

The treated product obtained in (a), in the above-mentioned (b), may be used as it is as a "cell wall lysing enzyme-decomposition product of yeast cell body residue" including the residue (insoluble fraction), or if necessary, those obtained by further subjecting to purification (for example, HPLC, ultrafiltration, etc.), concentration (for example, air drying, filtration under reduced pressure, etc.), sterilization (for example, heat sterilization, filtration sterilization, etc.), drying (for example, air drying, heating, reduced pressure, spray drying, freeze drying, etc.), etc., may be used as a "cell wall lysing enzyme-decomposition product of yeast cell body residue". Incidentally, the conditions in these steps can be appropriately adjusted by those skilled in the art.

The cell wall lysing enzyme-decomposition product of yeast cell body residue of the present invention contains lipid. The "lipid" used in the present specification is not particularly limited as long as it is, for example, simple lipids such as mono-, di-, and tri-acylglycerols, etc.; complex lipids such as glycolipids, phospholipids, etc.; and derived lipids which are oil-soluble fractions of these hydrolysates, and is a lipid originally contained in yeast (yeast-derived lipid). Typically, the lipid in the cell wall lysing enzyme-decomposition product of yeast cell body residue of the present invention contains phospholipids.

An amount of the lipid contained in the cell wall lysing enzyme-decomposition product of yeast cell body residue is not particularly limited as long as it is about 5% by mass or more based on the dry mass of the decomposition product, preferably about 5% by mass or more and about 15% by mass or less, and more preferably about 5% by mass or more and about 10% by mass or less.

The cell wall lysing enzyme-decomposition product of yeast cell body residue of the present invention may contain RNA. An amount of the RNA contained in the cell wall lysing enzyme-decomposition product of yeast cell body residue is preferably less than about 5% by mass, and more preferably less than about 4% by mass based on the dry mass of the decomposition product.

The cell wall lysing enzyme-decomposition product of yeast cell body residue of the present invention may contain amino acid(s). The "free amino acid" used in the present specification refers to an amino acid that exists in a free state without being bonded with other amino acids to form a peptide. As the free amino acid, whereas it is not limited to these, there may be mentioned, for example, isoleucine, leucine, valine, histidine, lysine, methionine, phenylalanine, threonine, tryptophan, asparagine, aspartic acid, alanine, arginine, cysteine, glutamine, glutamic acid, glycine, proline, serine and tyrosine, etc. These may be used alone or in combination of two or more kinds. Free amino acids may be either of L-isomer, D-isomer or DL-isomer.

An amount of the free amino acid contained in the cell wall lysing enzyme-decomposition product of yeast cell body residue is preferably about 21% by mass or less, more preferably about 12% by mass or less, and further preferably about 7% by mass or less based on the dry mass of the decomposition product.

The cell wall lysing enzyme-decomposition product of yeast cell body residue of the present invention contains peptides, and particularly not only soluble peptides but also insoluble peptides. The "peptides" used in the present specification refers to a material in which two or more amino acids are bound by peptide bonds, and proteins, etc., are also contained.

An amount of the peptides contained in the cell wall lysing enzyme-decomposition product of yeast cell body residue is preferably about 30% by mass or more, more preferably about 30% by mass or more and about 70% by mass or less, and further preferably about 40% by mass or more and about 60% by mass or less based on the dry mass of the decomposition product.

In one embodiment of the present invention, the above-mentioned decomposition product contains insoluble peptides. In the present invention, the "insoluble peptide" means a peptide contained in the residue (water-insoluble fraction or heavy liquid after centrifugation) after extraction treatment or enzyme treatment. An amount of the insoluble peptide contained in the decomposition product is preferably about 20% by mass or more, more preferably about 20% by mass or more about 60% by mass or less, and further preferably about 30% by mass or more about 50% by mass or less based on the dry mass of the decomposition product.

In one embodiment of the present invention, the decomposition product is a material obtained by the method containing the following:

(a) treating a yeast cell body residue with a cell wall lysing enzyme (preferably glucanase) at about 40 to about 60°C for about 1 to about 24 hours, and
(b) recovering the treated product obtained in (a).

In one embodiment of the present invention, it is provided a method for producing a composition containing a decomposition product of a yeast cell wall, which method contains

(A) treating a yeast cell body residue with a cell wall lysing enzyme, and
(B) recovering the treated product obtained in (A).

Specific embodiments of the steps (A) and (B) have the same meanings as mentioned in the above-mentioned steps (a) and (b).

The "composition containing a decomposition product of a yeast cell wall" of the present invention can be applied to the food composition, etc., as an emulsifier or an emulsion stabilizer for food, or a milk-like substitute. For example, the "composition containing a decomposition product of a yeast cell wall" of the present invention may be formulated in the food composition, etc., or mixed with the food composition before cooking, during cooking and/or after cooking. It may be adjusted to an appropriate concentration (for example, 1 to 20%), and used as it is for drinking as yeast milk (Yeast based milk), or may be used as a milk substitute during cooking. It becomes a new milk substitute that contains protein as a main component, suitably contains oils and fats, can maintain a turbid emulsified state and does not use animals or plants.

In one embodiment of the present invention, there is provided a food composition containing the "composition containing a decomposition product of a yeast cell wall" of the present invention, oils and fats and carbohydrates Such a food composition can be used as a powdered milk substitute, for example, in foods such as gratin, confectionery, etc.

The "oils and fats" used in the present specification may be mentioned, whereas it is not limited to these, vegetable oils and fats, animal oils and fats, processed oils and fats, for example, edible safflower oil, edible grape oil, edible soybean oil, edible sunflower oil, edible corn oil, edible cottonseed oil, sesame oil, edible rapeseed oil, edible rice bran oil, edible peanut oil, edible olive oil, edible palm oil, edible palm olein, edible palm stearin, edible palm kernel oil, edible coconut oil, edible blended oil, flavored edible oil, beef tallow, lard, chicken oil, fish oil, milk fat, hydrogenated oils and fats, etc.

The "carbohydrates" used in the present specification may be mentioned, for example, monosaccharides, disaccharides, trisaccharides, tetrasaccharides, oligosaccharides, polysaccharides, starch decomposition product, reduced starch decomposition product, etc., these may be used alone or in combination of two or more kinds. As the carbohydrates, whereas it is not limited to these, there may be mentioned, for example, ketotriose, aldotriose, erythrulose, ribulose, xylulose, lyxose, deoxyribose, psicose, fructose, sorbose, tagatose, allose, altrose, glucose, mannose, gulose, idose, galactose, talose, fucose, fuculose, rhamnose, sedoheptulose, sucrose, lactose, maltose, trehalose, turanose, cellobiose, raffinose, maltotriose, acarbose, stachyose, fructo-oligosaccharides, galacto-oligosaccharides, mannan oligosaccharides, glycogen, starch, cellulose, dextrin, corn syrup, corn syrup solid, glucan, etc., and these may be used alone or in combination of two or more kinds.

The food composition of the present invention may further contain, whereas it is not limited to these, for example, additives such as excipients, lubricants, binders, disintegrants, pH adjusters, solvents, solubilizers, suspending agents, buffers, preservatives, antioxidants, colorants, sweeteners, surfactants, etc. These additives can be used, for example, those conventionally known, and the amount to be used, etc., can be appropriately adjusted by those skilled in the art according to the purpose.

Or else, as the food composition to which the "composition containing a decomposition product of a yeast cell wall" of the present invention can be applied or contains, whereas it is not limited to these, there may be mentioned, for example, beverages, soups, dressings, semi-solid dressings, mayonnaise, confectionery, chocolate, margarine, fat spreads, ice cream, lacto-ice cream, ice milk, bread, retort foods, extract seasonings, processed oils and fats, cheese, cheese food, processed cheese and other processed foods, etc., each of which contains an emulsifier, an emulsion stabilizer or powdered milk or is planning to be contained.

The application amount of the "composition containing a decomposition product of a yeast cell wall" of the present invention is not particularly limited as long as the object of the present invention can be achieved. It can be added to the total amount of the food composition, etc., or the processed product thereof, for example, about 0.001 to about 30% by mass, preferably about 0.01 to about 20% by mass, and more preferably about 0.1 to about 15% by mass, as the cell wall lysing enzyme-decomposition product of yeast cell body residue (dry mass).

The "composition containing a decomposition product of a yeast cell wall" of the present invention can be applied to perfumery and cosmetics or quasi-drugs, etc., as an emulsifier, an emulsion stabilizer, or as a milk-like substitute. For example, the "composition containing a decomposition product of a yeast cell wall" of the present invention may be formulated into perfumes, cosmetics, skin care products, hair care products, toiletry products, etc.

EXAMPLES

Hereinafter, the present invention will be explained in more detail by referring to Examples, but these Examples do not intend to limit the scope of the present invention.

[Example 1]

Dried yeast (Hyper Yeast HG-DY available from Asahi Group Foods, Ltd.) was extracted with hot water and centrifuged to obtain water-insoluble fraction, which was dried to obtain a yeast cell body residue. A suspension (16%, in water) of the obtained yeast cell body residue was sterilized (121°C for 20 minutes) in an autoclave. To the sterilized suspension was added 0.5% by mass of glucanase (Denazyme GEL1/R, available from NAGASE & Co., Ltd.) based on the dry mass of the yeast cell body residue under the conditions of 50°C and pH 5.3, and treated at 50°C for 18 hours. Thereafter, the treated product was made about 80°C to deactivate the glucanase, concentrated to 30% and spray-dried to obtain a composition of Example 1.

[Example 2]

Dried yeast (Hyper Yeast HG-DY available from Asahi Group Foods, Ltd.) was extracted with hot water and centrifuged to obtain water-insoluble fraction, which was dried to obtain a yeast cell body residue. A suspension (16%, in water) of the obtained yeast cell body residue was sterilized (121°C for 20 minutes) in an autoclave. To the sterilized suspension was added 0.5% by mass of glucanase (FILTRASE (Registered Trademark) BR-XI, (available from DSM Japan)) based on the dry mass of the yeast cell body residue under the conditions of 50°C and pH 5.3, and treated at 50°C for 24 hours. Thereafter, the treated product was made about 80°C to deactivate the glucanase, concentrated to 30% and spray-dried to obtain a composition of Example 2.

[Comparative Example 1]

A hot water extract (Hyper Meast HG-Ps available from Asahi Group Foods, Ltd.) of dried yeast was made a composition of Comparative Example 1. Incidentally, the composition of Comparative Example 1 is a material in which dried yeast (Hyper Yeast HG-DY available from Asahi Group Foods, Ltd.) is extracted with hot water, and then the supernatant obtained by centrifugation is concentrated.

[Comparative Example 2]

Dried yeast (Hyper Yeast HG-DY available from Asahi Group Foods, Ltd.) was extracted with hot water, and the heavy liquid (insoluble fraction) obtained by centrifugation was recovered and spray-dried to make it a composition of Comparative Example 2.

Test Example 1: Evaluation of emulsifying ability

Edible rapeseed oil, the composition of Example 1, Example 2, Comparative Example 1 or Comparative Example 2, corn syrup solid, and water were mixed, and emulsified (8,000 rpm, 10 minutes) by a homogenizer (TK Homomixer, PRIMIX Corporation). The obtained each emulsified Dosage formulation was well stirred, then 100 mL of each Dosage formulation in a uniform state was allowed to stand at 25°C for 3 hours in a 100 mL graduated cylinder, and the emulsified (separated) state was evaluated by the position of the separation line. The compositions and the results of each Dosage formulation are shown in the following Table 1.

[Table 1]

| | | Dosage formulation 1 | Dosage formulation 2 | Dosage formulation 3 | Dosage formulation 4 |
|---|---|---|---|---|---|
| Composition | Edible rapeseed oil | 30g | 30g | 30g | 30g |
| | Example 1 | 5g | - | - | - |
| | Example 2 | - | 5g | - | - |
| | Comparative Example 1 | - | - | 7.7g[※1] | - |
| | Comparative Example 2 | - | - | - | 5g |
| | Corn syrup solid | 65g | 65g | 65g | 65g |
| | Water | 100g | 100g | 97.3g | 100g |
| Evaluation (Position of separation line) | | 0mL | 0mL | 62mL | 46mL |

*1: Corresponding to 5 g of solid component

It was confirmed that Dosage formulation 1 and Dosage formulation 2 using the compositions of Example 1 and Example 2 were emulsified without separation (position of separation line 0 mL). No precipitation of insoluble peptide was observed.

Test Example 2: Use of emulsifier substitute and emulsion stabilizer substitute, or production of milk-like substitute

In accordance with the composition shown in the following Table 2, raw materials were mixed and subjected to preliminary emulsification by a homogenizer (TK Homomixer, PRIMIX Corporation, 5,000 rpm, 10 minutes), and emulsified by a homogenizer (Homogenizer L-100-H2-CH, Sanwa Engineering Co., Ltd.: 45 pressure, 3 times) and the obtained emulsified product was spray-dried to obtain each Dosage formulation as a powder. Incidentally, Dosage formulations 5 and 11 are formulations that can be used as, for example, milk substitutes in foods such as gratin, confectionery, etc. In general, if emulsification of Dosage formulation containing oils and fats is incomplete, powderization by spray drying is difficult, but a milk-like substitute (powder) similar to Dosage formulation 5 using existing emulsifier (sorbitan fatty acid ester) and emulsion stabilizer (sodium caseinate), and vegetable oils and fats could be obtained in Dosage formulation 6 in which the existing emulsion stabilizer (sodium caseinate) had been replaced with the composition of Example 1, in Dosage formulation 7 in which the existing emulsifier (sorbitan fatty acid ester) had been replaced with the composition of Example 1 and in Dosage formulations 8 to 10 in which the existing emulsion stabilizer (sodium caseinate) and emulsifier (sorbitan fatty acid ester) had been replaced with the composition of Example 1. In addition, a milk-like substitute (powder) similar to Dosage formulation 11 using the existing emulsifier (sorbitan fatty acid ester) and emulsion stabilizer (sodium caseinate), and animal oils and fats could be also obtained in Dosage formulation 12 in which the existing emulsion stabilizer (sodium caseinate) and emulsifier (sorbitan fatty acid ester) had been replaced with the composition of Example 1. According to the above, it was demonstrated that the composition of the present invention can be used as emulsifier substitutes and emulsifier stabilizer substitutes, and also, milk-like substitutes can be produced by using the composition of the present invention.

[Table 2]

| Composition | Dosage formulation 5 | Dosage formulation 6 | Dosage formulation 7 | Dosage formulation 8 | Dosage formulation 9 | Dosage formulation 10 | Dosage formulation 11 | Dosage formulation 12 |
|---|---|---|---|---|---|---|---|---|
| Corn syrup solid | 60.2g | 52.2g | 60.2g | 53.2g | 59.2g | 65.0g | - | - |
| Edible coconut oil (hardened oil) | 33.0g | 33.0g | 33.0g | 33.0g | 33.0g | 30.0g | - | - |
| Whole milk powder | - | - | - | - | - | - | 4.0g | 4.0g |
| Cream | - | - | - | - | - | - | 52.4g | 52.4g |
| Butter | - | - | - | - | - | - | 4.0g | 4.0g |
| Glucose | - | - | - | - | - | - | 2.0g | 2.0g |
| Lactose | - | - | - | - | - | - | 29.6g | 21.6g |
| Casein Na | 3.0g | - | 3.0g | - | - | - | 6.0g | - |
| Sorbitan fatty acid ester | 1.0g | 1.0g | - | - | - | - | 2.0g | - |
| Example 1 | - | 11.0g | 1.0g | 11.0g | 5.0g | 5.0g | - | 16.0g |
| pH adjusting agent (phosphate) | 2.8g | 2.8g | 2.8g | 2.8g | 2.8g | - | - | - |
| Water | 100g | 100g | 100g | 100g | 100g | 100g | 100g | 100g |
| Total | 200g | 200g | 200g | 200g | 200g | 200g | 200g | 200g |

[Example 3]

Beer yeast was subject to self-digestion treatment, and the water-insoluble fraction obtained by centrifugation was dried to obtain a yeast cell body residue (Product name: yeast cell wall, available from Asahi Group Foods, Ltd.). A suspension (16%, in water) of the obtained yeast cell body residue was sterilized by a UHT sterilization (Ultra High Temperature Sterilization; ultra-high-temperature instantaneous sterilization) device (at 125°C for 40 seconds). To the sterilized suspension was added 0.2% by mass of glucanase (Denazyme GEL1/R, available from NAGASE & Co., Ltd.) based on the dry mass of the yeast cell body residue under the conditions of 50°C at pH 5.3, and treated at 50°C for 24 hours. Thereafter, the treated product was made about 80°C to deactivate the glucanase, sterilized by a UHT sterilization device (at 125°C for 40 seconds), and spray-dried to obtain a composition of Example 3.

[Comparative Example 3]

Self-digestion treated extract of beer yeast (Meast powder N, available from Asahi Group Foods, Ltd.) was made a composition of Comparative Example 3. Incidentally, the composition of Comparative Example 3 is a material obtained by subjecting beer yeast (available from Asahi Group Foods, Ltd.) to self-digestion treatment, and the supernatant obtained by centrifugation was concentrated and spray-dried.

[Comparative Example 4]

The heavy liquid (insoluble fraction) obtained by subjecting beer yeast (available from Asahi Group Foods, Ltd.) to self-digestion treatment, and centrifuged was recovered and spray-dried (Product name: yeast cell wall, available from Asahi Group Foods, Ltd.) was made a composition of Comparative Example 4.

Test Example 3: Evaluation of emulsifying ability

Edible rapeseed oil, the composition of Example 3, Comparative Example 3 or Comparative Example 4, corn syrup solid, and water were mixed, and emulsified (8,000 rpm, 10 minutes) by a homogenizer (TK Homomixer, PRIMIX Corporation). The obtained each emulsified Dosage formulation was well stirred, then 100 mL of each Dosage formulation in a uniform state was allowed to stand at 25°C for 3 hours in a 100 mL graduated cylinder, and the emulsified (separated) state was evaluated by the position of the separation line. The compositions and the results of each Dosage formulation are shown in the following Table 3.

[Table 3]

| | | Dosage formulation 13 | Dosage formulation 14 | Dosage formulation 15 |
|---|---|---|---|---|
| Composition | Edible rapeseed oil | 30g | 30g | 30g |
| | Example 3 | 5g | - | - |
| | Comparative Example 3 | - | 5g | - |
| | Comparative Example 4 | - | - | 5g |
| | Corn syrup solid | 65g | 65g | 65g |
| | Water | 100g | 100g | 100g |
| Evaluation (Position of separation line) | | 0mL | 76mL | 11mL |

It was confirmed that Dosage formulation 13 using the composition of Example 3 was emulsified without separation (position of separation line 0 mL). No precipitation of insoluble peptide was observed.

[Example 4]

The water-insoluble fraction obtained by extracting beer yeast with hot water and centrifuged was dried to obtain a yeast cell body residue. A suspension (16%, in water) of the obtained yeast cell body residue was sterilized (121°C, 15 minutes) in an autoclave. To the sterilized suspension was added 0.2% by mass of glucanase (Denazyme GEL1/R, available from NAGASE & Co., Ltd.) based on the dry mass of the yeast cell body residue under the conditions of 50°C at pH 5.3, and treated at 50°C for 24 hours. Thereafter, the treated product was made about 80°C to deactivate the glucanase, sterilized (121°C, 15 minutes) in an autoclave, and freeze-dried to obtain a composition of Example 4.

[Comparative Example 5]

Extract of beer yeast by hot water extraction was made a composition of Comparative Example 4. Incidentally, the composition of Comparative Example 5 is a material in which the supernatant obtained by extracting beer yeast (available from Asahi Group Foods, Ltd.) with hot water and centrifuged is concentrated and spray-dried.

[Comparative Example 6]

A material (available from Asahi Group Foods, Ltd.) in which the heavy liquid (insoluble fraction) obtained by extracting beer yeast (available from Asahi Group Foods, Ltd.) with hot water and centrifuged was recovered and spray-dried was made a composition of Comparative Example 6.

Test Example 4: Evaluation of emulsifying ability

Edible rapeseed oil, the composition of Example 4, Comparative Example 5 or Comparative Example 6, corn syrup solid, and water were mixed, and emulsified (8,000 rpm, 10 minutes) by a homogenizer (TK Homomixer, PRIMIX Corporation). The obtained each emulsified Dosage formulation was well stirred, then 100 mL of each Dosage formulation in a uniform state was allowed to stand at 25°C for 3 hours in a 100 mL graduated cylinder, and the emulsified (separated) state was evaluated by the position of the separation line. The compositions and the results of each Dosage formulation are shown in the following Table 4.

[Table 4]

| | | Dosage formulation 16 | Dosage formulation 17 | Dosage formulation 18 |
|---|---|---|---|---|
| Composition | Edible rapeseed oil | 30g | 30g | 30g |
| | Example 4 | 5g | - | - |
| | Comparative Example 5 | - | 5g | - |
| | Comparative Example 6 | - | - | 5g |
| | Corn syrup solid | 65g | 65g | 65g |
| | Water | 100g | 100g | 100g |
| Evaluation (Position of separation line) | | 0mL | 11mL | 47mL |

It was confirmed that Dosage formulation 16 using the composition of Example 4 was emulsified without separation (position of separation line 0 mL). No precipitation of insoluble peptide was observed.

Test Example 5: Composition analysis

Amounts of insoluble peptide and lipid contained in the composition of Example 1 were analyzed. The results are shown in Table 5.

[Table 5]

| Composition | Example 1 (% by mass) |
|---|---|
| Insoluble peptide | 45.3 |
| Lipid | 7.2 |

Incidentally, the analyzing method of insoluble peptides and lipids, and the calculation method of the contained ratio of each component are as follows. Incidentally, unless otherwise specifically mentioned, the contained ratio means % by mass.

<Insoluble peptide>

The contained ratio of the insoluble peptide is calculated by subtracting the value obtained by multiplying the protein contained ratio of the water-soluble fraction of the composition of Example 1 by the solubilization ratio of the composition of Example 1, from the protein contained ratio of the composition of Example 1. Here, the solubilization ratio (supernatant) in the present invention can be calculated by the following equation (1).
[Numerical formula 1]

$$\text{Solubilization ratio (\%)} = \text{Mass B of solid component/Mass A of solid component} \times 100 \quad \cdots \text{Formula (1)}$$

In the above-mentioned equation (1), "Mass A of solid component" indicates a mass of the dried product obtained by drying X g of the subject sample at 105°C for 5 hours.

In addition, in the above-mentioned equation (1), "Mass B of solid component" indicates a mass of the dried product obtained by centrifuging X g of the subject sample at 5,000 G for 5 minutes, and drying the obtained supernatant at 105°C for 5 hours.

Protein was measured by the combustion method (modified Duma's method). The protein contained ratio of the composition of Example 1 was 51.6%, and the protein contained ratio (Mass B of solid component) of the soluble fraction of the composition of Example 1 was 16.1%. In addition, the solubilization ratio (supernatant) of the composition of Example 1 was 39%.

<Lipid>

Lipid was measured by the Soxhlet extraction method and calculated as a ratio (%) to the dry mass of the composition.

Test Example 6: Use of emulsifier substitute and emulsion stabilizer substitute

In accordance with the composition shown in the following Table 6, raw materials other than brewed vinegar were mixed, and after heating to 80°C for 10 minutes, brewed vinegar was mixed at 70°C or lower, subjected to preliminary emulsification by a homogenizer (TK Homomixer, PRIMIX Corporation, 8,000 rpm, 10 minutes), and emulsified by a homogenizer (Homogenizer L-100-H2-CH, Sanwa Engineering Co., Ltd.: 45 pressure and 3 times) to obtain each Dosage formulation 19 and 20 as dressing state. Incidentally, Dosage formulation 19 was not emulsified and separated immediately after the processing. It was confirmed that Dosage formulation 20 in which dextrin in Dosage formulation 19 had been replaced with the composition of Example 1 maintained an emulsified state for a certain period of time (about 2 weeks) at normal temperature. According to this, it was shown that the composition of the present invention could be used as an emulsifier substitute and an emulsion stabilizer substitute.

[Table 6]

| Composition | Dosage formulation 19 | Dosage formulation 20 |
|---|---|---|
| Edible rapeseed oil | 31.0g | 31.0g |
| Corn syrup | 30.0g | 30.0g |
| Sugar | 3.0g | 3.0g |
| Brewed vinegar | 8.0g | 8.0g |
| Onion powder | 2.5g | 2.5g |
| Salt | 5.5g | 5.5g |
| Dextrin | 5.0g | - |
| Example 1 | - | 5.0g |
| Water | 15.0g | 15.0g |
| Total | 100g | 100g |

Test Example 7: Use of emulsifier substitute and emulsion stabilizer substitute

In accordance with the composition shown in the following Table 7, raw materials were mixed and after heating at 70°C for 1 minute, subjected to preliminary emulsification by a homogenizer (TK Homomixer, PRIMIX Corporation, 8,000 rpm, 10 minutes), and emulsified by a homogenizer (Homogenizer L-100-H2-CH, Sanwa Engineering Co., Ltd.: 45 pressure, 3 times) to obtain Dosage formulation 21 and 22 as a vegetable oils and fats cream. Incidentally, Dosage formulation 21 was not emulsified and separated immediately after the processing. It was confirmed that Dosage formulation 22 in which dextrin of Dosage formulation 21 had been replaced with the composition of Example 1 maintained an emulsified state for a certain period of time (about 1 weeks) under refrigeration (10°C or lower). According to this, it was shown that the composition of the present invention could be used as an emulsifier substitute and an emulsion stabilizer substitute.

[Table 7]

| Composition | Dosage formulation 21 | Dosage formulation 22 |
|---|---|---|
| Edible rapeseed oil | 20.0g | 20.0g |
| Edible coconut oil (hardened oil) | 20.0g | 20.0g |
| Dextrin | 6.0g | - |
| Example 1 | - | 6.0g |
| Water | 54.0g | 54.0g |
| Total | 100g | 100g |

Test Example 8: Use of milk-like substitute

In accordance with the composition shown in the following Table 8, raw materials were mixed and emulsified by a homogenizer (Homogenizer LAB 1000, SMT Co., Ltd.: 45 pressure, 2 times) to obtain Dosage formulation 23 and Dosage formulation 24 as milk-like substitutes. It was confirmed that Dosage formulation 23 maintained an emulsified state for a certain period of time (about 3 days) at normal temperature. Incidentally, the composition of Dosage formulation 23 was prepared by referring to the composition of cow's milk and preparing a composition with the same oil content and non-fat solid content as cow's milk. According to this, it was shown that the composition of the present invention could be used as a milk-like substitute.

Table 8]

| Composition | Dosage formulation 23 | Dosage formulation 24 |
|---|---|---|
| Edible rapeseed oil | 3.8g | - |
| Example 1 | 8.8g | 8.8g |
| Water | 87.4g | 91.2g |
| Total | 100g | 100g |

UTILIZABILITY IN INDUSTRY

As mentioned above, as shown in Examples, according to the present invention, it is possible to provide a composition containing a decomposition product of a yeast cell wall useful as an emulsifier substitute, an emulsion stabilizer substitute or a milk-like substitute using a yeast cell body residue which is inexpensive and easily available as a raw material. In addition, the composition of the present invention can be easily produced by decomposing the yeast cell body residue with the cell wall lysing enzyme. These compositions are derived from non-animal raw material, do not require indication of food additives, and are free from allergens such as milk, eggs and legumes, so that it can be expected to be used, as an emulsifier or an emulsion stabilizer, or as a milk-like substitute, for various kinds of foods.

**Claims**

1.  A composition containing a decomposition product of a yeast cell wall which comprises a cell wall lysing enzyme-decomposition product of yeast cell body residue.

2.  A composition containing a decomposition product of a yeast cell wall comprising a cell wall lysing enzyme-decomposition product of yeast cell body residue, wherein the decomposition product contains 5% by mass or more of lipid based on a dry mass of the decomposition product.

3. The composition according to Claim 1 or 2, wherein an RNA content of the decomposition product is less than 5% by mass based on a dry mass of the decomposition product.

4. The composition according to any one of Claims 1 to 3, wherein the decomposition product contains an insoluble peptide.

5. The composition according to any one of Claims 1 to 4, wherein the cell wall lysing enzyme is glucanase.

6. The composition according to any one of Claims 1 to 5, wherein the cell wall lysing enzyme is glucanase having a $\beta$-1,3, $\beta$-1,4, and/or $\beta$-1,6 activity.

7. The composition according to any one of Claims 1 to 6, wherein the cell wall lysing enzyme is glucanase derived from Streptomyces.

8. The composition according to any one of Claims 1 to 7, wherein the cell wall lysing enzyme is glucanase having no protease activity.

9. The composition according to any one of Claims 1 to 8, wherein the lipid contains at least yeast-derived phospholipid.

10. The composition according to any one of Claims 1 to 9, wherein the decomposition product contains 5% by mass or more and 15% by mass or less of lipid based on a dry mass of the decomposition product.

11. The composition according to any one of Claims 1 to 10, wherein the decomposition product is a material obtained by the method containing the following:

    (a) treating a yeast cell body residue with glucanase at 40 to 60°C for 1 to 24 hours, and
    (b) recovering the treated product obtained in (a).

12. The composition according to any one of Claims 1 to 11, wherein the yeast cell body residue is a residue of yeast cell body after extraction of yeast with hot water.

13. A method for producing a composition containing a decomposition product of a yeast cell wall, which comprises

    (A) treating a yeast cell body residue with a cell wall lysing enzyme, and
    (B) recovering the treated product obtained in (A).

14. An emulsifier for food or an emulsion stabilizer for food which comprises the composition according to any one of Claims 1 to 12.

15. A food composition which comprises the composition according to any one of Claims 1 to 12, oils and fats, and carbohydrates.

16. Use of the composition according to any one of Claims 1 to 12 as an emulsifier for food or an emulsion stabilizer for food.

17. Use of the composition according to any one of Claims 1 to 12 as a milk-like substitute.

## INTERNATIONAL SEARCH REPORT

| International application No. |
| --- |
| **PCT/JP2022/002006** |

**A. CLASSIFICATION OF SUBJECT MATTER**

*C09K 23/00*(2022.01)i; *A23L 27/60*(2016.01)i; *A23C 11/00*(2006.01)i; *A23L 5/00*(2016.01)i; *A23L 29/10*(2016.01)i; *A23L 33/145*(2016.01)i

FI:    A23L33/145; A23L5/00 L; A23C11/00; B01F17/00; A23L27/60 A; A23L29/10

According to International Patent Classification (IPC) or to both national classification and IPC

**B. FIELDS SEARCHED**

Minimum documentation searched (classification system followed by classification symbols)

C09K23/00; A23L5/00; A23L27/60; A23L29/10; A23L33/145; A23C11/00

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/MEDLINE/EMBASE/BIOSIS/CABA/FSTA (STN); JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| X | LI, Jin et al. Characterization of the composition and the techno-functional properties of mannnoproteins from Saccharomyces cerevisiae yeast cell walls, Food Chemistry, 22 May 2019, 297, 124867, DOI:10.1016/j.foodchem.2019.05.141<br>2.2.2, 2.5.3, 3.2.3 | 1, 3-8, 11-17 |
| X | CAMERON, David R. et al. The Mannnoprotein of Saccharomyces cerevisiae Is an Effective Bioemulsifier. Applied and Environmental Microbiology , 1988, 54(6), 1420-1425<br>Materials and Methods, Results, Discussion | 1-17 |
| X | JP 61-227827 A (SAPPORO BREWERIES LTD.) 09 October 1986 (1986-10-09)<br>claims, examples | 1-17 |
| X | JP 4-58893 A (ASAHI BREWERIES, LTD.) 25 February 1992 (1992-02-25)<br>claims, examples | 1-13, 15, 17 |
| X | JP 2002-209598 A (KIRIN BREWERY CO., LTD.) 30 July 2002 (2002-07-30)<br>claims 1-17, examples | 1-13, 15, 17 |

☑ Further documents are listed in the continuation of Box C.     ☑ See patent family annex.

| | |
| --- | --- |
| *    Special categories of cited documents:<br>"A"  document defining the general state of the art which is not considered to be of particular relevance<br>"E"  earlier application or patent but published on or after the international filing date<br>"L"  document which may throw doubts on priority claim(s) or which is cited to establish the publication date of another citation or other special reason (as specified)<br>"O"  document referring to an oral disclosure, use, exhibition or other means<br>"P"  document published prior to the international filing date but later than the priority date claimed | "T"  later document published after the international filing date or priority date and not in conflict with the application but cited to understand the principle or theory underlying the invention<br>"X"  document of particular relevance; the claimed invention cannot be considered novel or cannot be considered to involve an inventive step when the document is taken alone<br>"Y"  document of particular relevance; the claimed invention cannot be considered to involve an inventive step when the document is combined with one or more other such documents, such combination being obvious to a person skilled in the art<br>"&"  document member of the same patent family |
| Date of the actual completion of the international search<br><br>**24 February 2022** | Date of mailing of the international search report<br><br>**08 March 2022** |
| Name and mailing address of the ISA/JP<br><br>**Japan Patent Office (ISA/JP)**<br>**3-4-3 Kasumigaseki, Chiyoda-ku, Tokyo 100-8915**<br>**Japan** | Authorized officer<br><br><br><br>Telephone No. |

Form PCT/ISA/210 (second sheet) (January 2015)

## INTERNATIONAL SEARCH REPORT

| | International application No. |
|---|---|
| | **PCT/JP2022/002006** |

### C. DOCUMENTS CONSIDERED TO BE RELEVANT

| Category* | Citation of document, with indication, where appropriate, of the relevant passages | Relevant to claim No. |
|---|---|---|
| X | JP 2020-078268 A (KOHJIN LIFE SCIENCES CO., LTD.) 28 May 2020 (2020-05-28)<br>claims 1-5, examples | 1-13, 15, 17 |
| X | JP 2018-531586 A (DSM IP ASSETS BV) 01 November 2018 (2018-11-01)<br>claims 1-17, examples | 1-13, 15, 17 |
| A | NEROME, Shinsuke et al. Cell surface changes that advance the application of using yeast as a food emulsifier, Food Chemistry, 21 January 2020, 315, 126264, DOI:10.1016/j.foodchem.2020.126264<br>whole document | 1-17 |
| A | JP 2008-271820 A (ASAHI BREWERIES, LTD.) 13 November 2008 (2008-11-13)<br>claims 1-17, examples | 1-17 |
| A | JP 2012-231747 A (KOHJIN CO., LTD.) 29 November 2012 (2012-11-29)<br>claims 1-4, examples | 1-17 |
| P, X | WO 2021/049619 A1 (ASAHI GROUP FOODS, LTD.) 18 March 2021 (2021-03-18)<br>claims 1-16, examples | 1-13, 15, 17 |
| P, X | WO 2021/140978 A1 (ASAHI GROUP FOODS, LTD.) 15 July 2021 (2021-07-15)<br>claims 1-14, examples | 1-13, 15, 17 |

Form PCT/ISA/210 (second sheet) (January 2015)

**INTERNATIONAL SEARCH REPORT**
Information on patent family members

International application No.

**PCT/JP2022/002006**

| Patent document cited in search report | | | Publication date (day/month/year) | Patent family member(s) | | | Publication date (day/month/year) |
|---|---|---|---|---|---|---|---|
| JP | 61-227827 | A | 09 October 1986 | (Family: none) | | | |
| JP | 4-58893 | A | 25 February 1992 | (Family: none) | | | |
| JP | 2002-209598 | A | 30 July 2002 | (Family: none) | | | |
| JP | 2020-078268 | A | 28 May 2020 | (Family: none) | | | |
| JP | 2018-531586 | A | 01 November 2018 | US<br>claims 1-17, examples<br>WO<br>CA<br>CN<br>BR | 2018/0271130<br><br>2017/050629<br>2997401<br>108024548<br>112018005411 | A1<br><br>A1<br>A<br>A<br>A | |
| JP | 2008-271820 | A | 13 November 2008 | (Family: none) | | | |
| JP | 2012-231747 | A | 29 November 2012 | (Family: none) | | | |
| WO | 2021/049619 | A1 | 18 March 2021 | (Family: none) | | | |
| WO | 2021/140978 | A1 | 15 July 2021 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012231747 A